(19) Europäisches Patentamt
European Patent Office
Office européen des brevets

(11) Publication number: **0 080 570**
**B1**

(12) **EUROPEAN PATENT SPECIFICATION**

(45) Date of publication of patent specification: **17.12.86**

(21) Application number: **82108776.4**

(22) Date of filing: **22.09.82**

(51) Int. Cl.⁴: **C 07 D 311/78,**
C 07 D 493/10, A 61 K 31/35
// (C07D493/10, 311:00, 307:00)

# (54) Ailanthone derivatives.

(30) Priority: **22.09.81 JP 151124/81**

(43) Date of publication of application:
**08.06.83 Bulletin 83/23**

(45) Publication of the grant of the patent:
**17.12.86 Bulletin 86/51**

(84) Designated Contracting States:
**AT BE CH DE FR GB IT LI LU NL SE**

(56) References cited:
**EXPERIENTIA, vol. 34, no. 9, 15-09-1978 BASEL (CH) J. POLONSKY: "The isolation and structure of 13,18-dehydroglau-carubinone, a new antineoplastic quassinoid from Simarouba amary", pages 1122-23**
**JOURNAL OF ORGANIC CHEMISTRY, vol. 40, no. 17, 22-08-1975 COLUMBUS OHIO (US) J. POLONSKY et al.: "Carbon-13 Nuclear Magnetic Resonance Spectral Analysis of Quassinoid Bitter Principles", pages 2499-2504**

**The file contains technical information submitted after the application was filed and not included in this specification**

(73) Proprietor: **Suntory Limited**
**1-40 Dojimahama 2-chome Kita-ku**
**Osaka-shi Osaka-fu 530 (JP)**

(72) Inventor: **Honda, Tadashi**
**c/o Suntory Ibaraki-Ryo No. 3-5, Shimochujyo-cho**
**Ibaraki-shi Osaka (JP)**
Inventor: **Imao, Kayoko**
**No. 28, Ikomadai-minami**
**Ikoma-shi Nara (JP)**
Inventor: **Nakatsuka, Nobuo**
**No. 21-5, Miyanosaka 3-chome**
**Hirakata-shi Osaka (JP)**
Inventor: **Nakanishi, Toshihiro**
**No. 16-16, Futaba-cho**
**Ibaraki-shi Osaka (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

This invention relates to novel antineoplastic ailanthone derivatives, and, more particulary, it relates to 15β-hydroxyailanthone-15-carboxylic acid esters represented by the following formulae IIa and IIb; to a method for preparing the compounds IIa and IIb; and to the use of the compound IIa and lib as an antitumor agent.

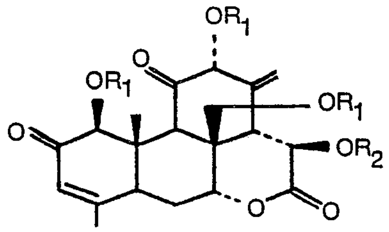

(IIa)

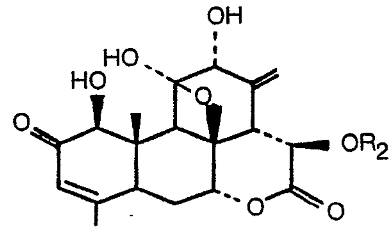

(IIb)

wherein $R_1$ is hydrogen, an acetyl, chloroacetyl or benzoyl group and $R_2$ represents a $C_5$—$C_{18}$ α,β-unsaturated straight chain acyl group.

Background of the Invention

For the past 30 years, many efforts were continuously made to find antineoplastic agents from natural sources and to synthesize their analogues. As the reuslt, a few of them have been clinically used as antitumor agent. However, effective antitumor agents generally have the defect of high toxicity. Therefore, there is nothing to be satisfied in view of chemotherapy against malignant tumors.

The publication by J. Polonsky et al. in Experientia, *13* (1978), pages 1122—23, describes 13,18-dehydroglaucarubinone to be effective against lymphocytic leukemia P388. Respective dehydroglaucarubinone comprises in the 15-position an α-hydroxyacyl group instead of a $C_5$—$C_{18}$ α,β-unsaturated straight acyl group according to the present invention. It is assumed that introduction of an α,β-unsaturated straight chain acyl group having an appropriate chain length according to the invention has a remarkable effect on the pharmacological activities of respective compounds.

Summary of the Invention

The present inventors have heretofore noticed to ailanthone (11β,20-epoxy-1β,11α,12α-trihydroxypicrasa-3,13 (21)-diene-2,16-dione) represented by the following formula (III), which is contained in the bark of "tree of heaven" (Japanese name "Shinju" or "Niwaurushi"; *Ailanthus altissima,* Swingle, Simarubaceae), and have synthesized its many derivatives for antineoplastic screening test.

Thus, the present invention concerns antineoplastic ailanthone derivatives represented by the following formula

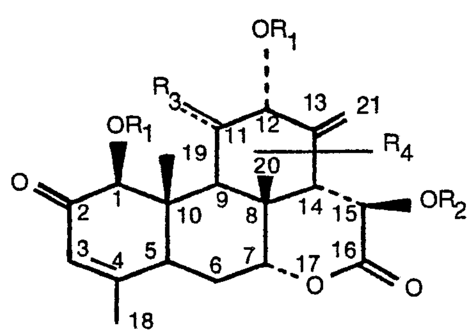

wherein $R_1$ is hydrogen, an acetyl, chloroacetyl, or benzoyl group, $R_2$ is a $C_5$—$C_{18}$ α,β-unsaturated straight chain acyl group, $R_3$ is oxygen or a hydroxyl group and $R_4$ is oxygen connecting to $C_{11}$—$C_{20}$ carbon atom, or acetyloxy, chloroacetyloxy, or benzoyloxy group connecting to $C_{20}$ carbon atom of the nucleus, with the proviso, that when $R_4$ is oxygen, then $R_3$ is a hydroxyl group, and that when $R_4$ is an acyloxy group as defined above, then $R_3$ is oxygen.

A preferred compound of the invention is represented by the following formula

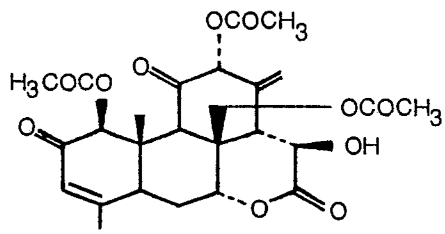

The compound IIa and IIb of the present invention can e.g. by synthesized from ailanthone III as the starting material, according to the following scheme:

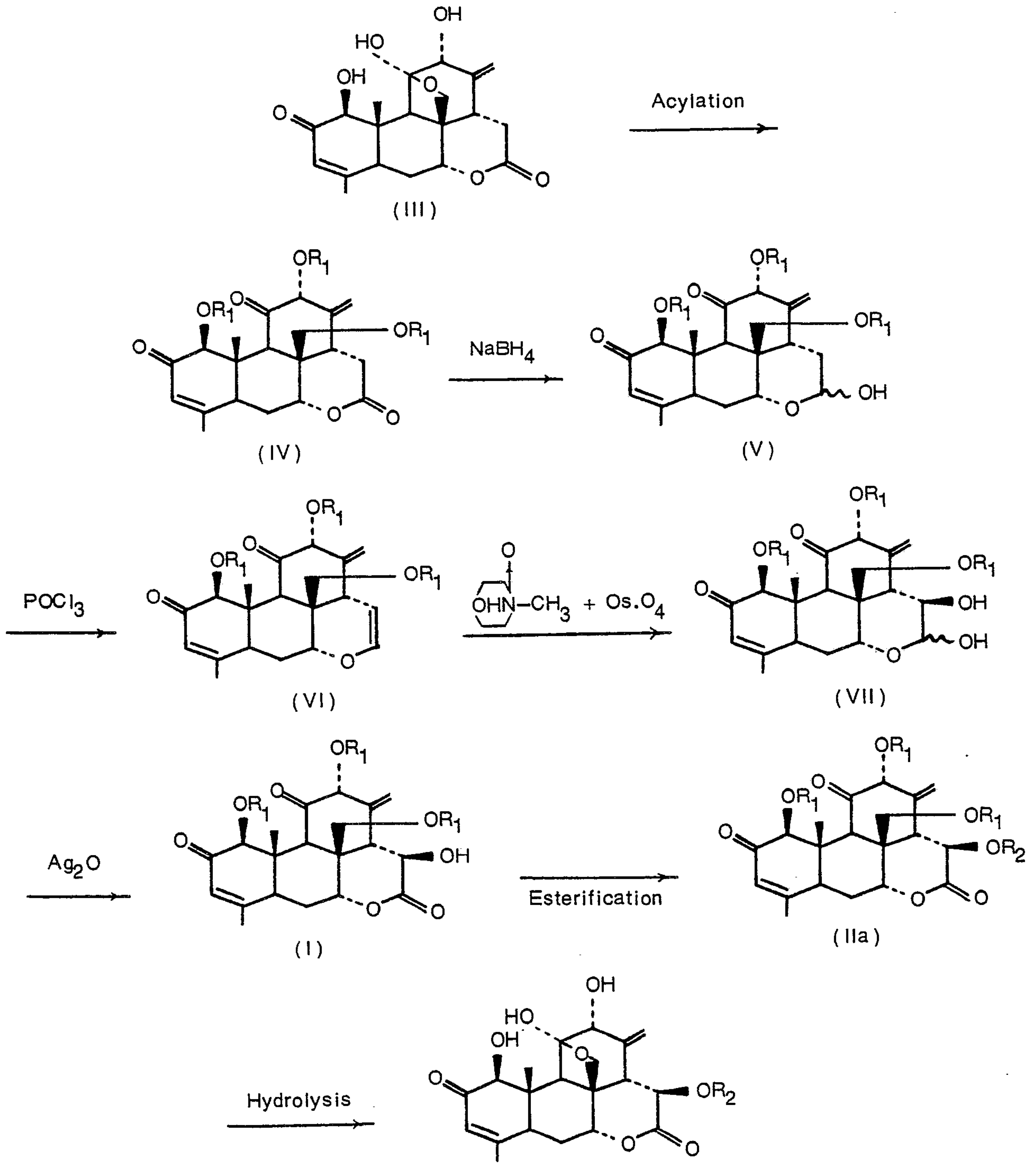


(III) (IV) (V) (VI) (VII) (I) (IIa) (IIb)

wherein $R_1$ is an acyl group as defined above and $R_2$ is a $C_5$—$C_{18}$ α,β-unsaturated straight chain acyl group.

That is, ailanthone III, which can be extracted and isolated from the bark of "tree of heaven" according to usual manner, is acylated as usual with an acylating agent such as acetic anhydride, acetyl chloride, benzoyl chloride or chloroacetyl chloride to give a 1,12,20-triacyloxy compound IV. During this transformation, C-11 hemiacetal bond is cleaved to give an 11-keto-20-alcohol.

Then, the above triacyloxy compound whose hydroxyl group is acylated IV is reduced with a selective reducing agent such as sodium borohydride thereby only C-16 ketone of the compound IV is selectively reduced to a secondary alcohol (V). This alcohol is a mixture of stereoisomers. Then, the alcohol V is dehydrated to the corresponding compound VI with a dehydrating agent such as phosphorylchloride or phosphorus pentoxide and this unsaturated compound VI is further oxidized with N-methylmorpholine-N-oxide and osmium tetroxide to the corresponding 15,16-dihydroxy compound VII. Then, the resulting compound VII can be converted to the 16-ketone compound I by mild oxidation with silver oxide.

Finally, the ketone I is esterified with a $C_5$—$C_{18}$ α,β-unsaturated straight carboxylic acid, in the presence of cesium fluoride and 1-ethyl-2-fluoropyridinium tetrafluoroborate or 1-methyl-2-fluoropyridinium tosylate to give the desired compound IIa. Moreover, the compound IIa is hydrolyzed with an alkali to give another desired compound IIb.

The substituent $R_2$ of the compound IIa or IIb is a $C_5$—$C_{18}$ α,β-unsaturated straight acyl group such as cis- or trans-2-pentenoyl, cis- or trans-2-hexenoyl, cis- or trans-2-heptenoyl, cis- or trans-2-octenoyl, cis- or trans-2-nonenoyl, cis- or trans-2-decenoyl, cis- or trans-2-undecenoyl, cis- or trans-2-dodecenoyl, cis- or trans-2-tridecenoyl, cis- or trans-2-tetradecenoyl, cis- or trans-2-pentadecenoyl, cis- or trans-2-hexadecenoyl, cis- or trans-2-octadecenoyl groups.

The esterification reaction to give the compound IIa can be carried out by several usual manners such as by reacting the compound I with an anhydride or a halide of a $C_5$—$C_{18}$ α,β-unsaturated straight carboxylic acid or with above acid in the presence of trifluoroacetone as a dehydrating agent. However, according to our experiments, the above reaction in the presence of 1-ethyl-2-fluoropyridinium tetrafluoroborate or 1-methyl-2-fluoropyridinium tosylate and cesium fluoride as a dehydrating agent appears to be preferable in view of the yield of the desired compound.

The compound IIa can be hydrolyzed to the compound IIb under a mild hydrolysis condition. If stonger condition is used, even C-15 ester *per se* is also hydrolyzed. Therefore, it is preferably that above transformation is carried out in an alcoholic solution of an alkali metal alkoxide for a long period of time.

Detailed Description of the Invention

(A) *Physico-chemical data of the Present Compounds*

(i) Compound I ($R_1$=$CH_3CO$—)

Form: colorless needles

mp: 231—232°C

Specific rotation (SR): $[\alpha]_D^{25}$ + 20.5° (c=0.20, $CHCl_3$)

Infrared spectrum (IR) (KBr, $cm^{-1}$): 3540, 1760, 1740, 1680

Ultraviolet absorption (UV) ($\lambda_{max}^{EtOH}$): 238 nm (ε = 9000), 203 nm (ε = 5400)

Proton NMR (NMR) ($CDCl_3$, δ ppm): 1.41, s (3H, 10-$CH_3$); 1.95, s (3H, 4-$CH_3$); 2.03, 2.08, 2.10, s (each 3H, 3x —$OCOCH_3$); 3.01, brd (1H, 5-H); 3.14, d, J=12Hz (1H, 14β-H); 3.18, d, J=2Hz (1-H, 15β-OH); 3.56, s, (1H, 9α-H);

3.56, d, J=12Hz (1H) }
4.52, d, J=12Hz (1H) } —$CH_2$—OAc;

4.69, t, (1H, 7β-H); 5.16, s, (1H, 12-H); 5.04, dd, J=2Hz, 12Hz (1H, 15α-H);

5.38, s (1H) }
5.54, s (1H) } =
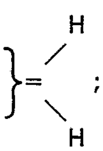
;

54.6, s (1H, 1-H) 6.04, q, (1H, 3-H)

Mass spectrum (MS) (m/e): $M^+$518, 476, 458, 416, 392, 374

| Elemental analysis: | C | H |
|---|---|---|
| Found | 60.27 | 5.80%; |
| Calcd. | 60.23 | 5.83%. |

X-ray diffraction analysis

R-value (0.071 except |FOBS|=0)

Crystallization Recrystallized from ethanol-water

Specific gravity 1.37

Space group $P2_1$ (monoclinic) Z=2 lattice constants

a = 10.254Å (σ(a) = 0.001)    c = 8,4670Å (σ(c) = 0.0009)

b = 14,560Å (σ(b) = 0.002)    β = 101.45° (σ(β) = 0.01°)

(ii) Compound IIa ($R_1=CH_3CO—$)
General formula

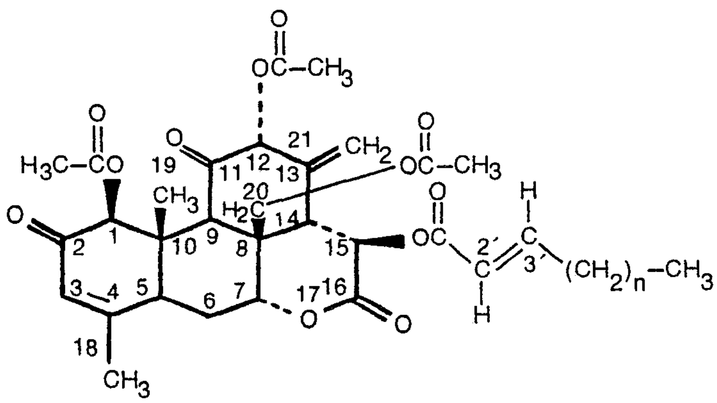

(1) n=14
Form: amorphous
SR: $[\alpha]_D^{24}$ −2.00° (c=0.15, $CHCl_3$)
IR (KBr, $cm^{-1}$): 1750, 1740, 1730, 1680
UV ($\lambda_{max}^{EtOH}$): 210 nm, 238 nm
NMR ($CDCl_3$, δ ppm): 0.90 (terminal —$CH_3$); 1.28 (5′-17′$CH_2$); 1.45 (3H, s, 10-$CH_3$); 1.97 (3H, s, 4-$CH_3$);

2.04, 2.10, 2.16 (3H, s, 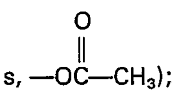);

3.12 (1H, brd, 5-H); 3.34 (1H, d, J=12Hz, 14-H); 3.58, 4.58 (1H, d, J=12Hz, —$CH_2$—O—); 3.72 (1H, s, 9-H); 4.74 (1H, t, 7-H); 5.26 (1H, s, 12-H); 5.42 (1H, s, 1-H);

5.30, 5.50 (1H, s, 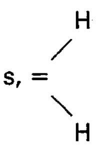);

5.86 (1H, dt, J=16, 1Hz, 2′-H); 6.06 (1H, brs, 3-H); 6.28 (1H, d, J=12Hz, 15-H); 7.08 (1H, dt, J=16, 7Hz, 3′-H)
MS (m/e): 782, 740, 698, 680, 638, 514, 490, 472

| Elemental analysis: | C | H |
|---|---|---|
| Found | 67.37 | 7.89% |
| Calcd. | 67.52 | 7.93% |

(2) n=13
Form: amorphous
IR (KBr, $cm^{-1}$): 1750, 1740, 1730, 1680
UV ($\lambda_{max}^{EtOH}$): 210 nm, 238 nm
NMR ($CDCl_3$, δ ppm): 1.28 (5′-16′$CH_2$)

| Elemental analysis: | C | H |
|---|---|---|
| Found | 67.01 | 7.79% |
| Calcd. | 67.19 | 7.81% |

(3) n=12
Form: amorphous
SR: $[\alpha]_D^{23}$ −4.35° (c=0.23, $CHCl_3$)
IR (KBr, $cm^{-1}$): 1760, 1750, 1730, 1680
UV ($\lambda_{max}^{EtOH}$): 210 nm, 238 nm
NMR ($CDCl_3$, δ ppm): 1.28 (5′-15′-$CH_2$)

| Elemental analysis: | C | H |
|---|---|---|
| Found | 66.71 | 7.73% |
| Calcd. | 66.84 | 7.69% |

(4) n=11

Form: amorphous

IR (KBr, $cm^{-1}$): 1760, 1740, 1730, 1680

UV ($\lambda_{max}^{EtOH}$): 210 nm, 238 nm

NMR ($CDCl_3$, δ ppm): 1.28 (5'-14'-$CH_2$—)

| Elemental analysis: | C | H |
|---|---|---|
| Found | 66.30 | 7.55% |
| Calcd. | 66.49 | 7.57% |

(5) n=10

Form: amorphous

SR: $[\alpha]_D^{24}$ −1.58° (c=0.19, $CHCl_3$)

IR (KBr, $cm^{-1}$): 1760, 1740, 1730, 1680

UV ($\lambda_{max}^{EtOH}$): 210 nm, 238 nm

NMR ($CDCl_3$, δ ppm): 1.28 (5'-13'-$CH_2$—)

MS (m/e): 726, 684, 642, 624, 582, 458, 434, 416

| Elemental analysis: | C | H |
|---|---|---|
| Found | 66.03 | 7.43% |
| Calcd. | 66.10 | 7.49% |

(6) n=9

Form: amorphous

SR: $[\alpha]_D^{24}$ −1.18° (c=0.17, $CHCl_3$)

IR (KBr, $cm^{-1}$): 1740, 1730, 1680

UV ($\lambda_{max}^{EtOH}$): 210 nm, 238 nm

NMR ($CDCl_3$, δ ppm): 1.28 (5'-12'-$CH_2$—)

MS (m/e): 712, 670, 628, 500, 476, 458

| Elemental analysis: | C | H |
|---|---|---|
| Found | 65.80 | 7.34%; |
| Calcd. | 65.71 | 7.35%. |

(7) n=8

Form: amorphous

SR: $[\alpha]_D^{24}$ −3.46° (c=0.26, $CHCl_3$)

IR (KBr, $cm^{-1}$): 1760, 1750, 1740, 1680

UV ($\lambda_{max}^{EtOH}$): 210 nm, 238 nm

NMR ($CDCl_3$, δ ppm): 1.28 (5'-11'-$CH_2$—)

MS (m/e): 698, 656, 638, 614, 596, 458, 434, 416

| Elemental analysis: | C | H |
|---|---|---|
| Found | 65.22 | 7.22%; |
| Calcd. | 65.21 | 7.21%. |

(8) n=7

Form: amorphous

SR: $[\alpha]_D^{27}$ −9.38° (c=0.16, $CHCl_3$)

IR (KBr, $cm^{-1}$): 1750, 1740, 1680

UV ($\lambda_{max}^{EtOH}$): 210 nm, 238 nm

NMR ($CDCl_3$, δ ppm): 1.28 (5'-10'-$CH_2$—)

MS (m/e): 684, 642, 600, 472, 448, 430

| Elemental analysis: | C | H |
|---|---|---|
| Found | 64.82 | 7.03%; |
| Calcd. | 64.91 | 7.02%. |

(9) n=6

Form: amorphous

SR: $[\alpha]_D^{27}$ −7.50° (c=0.20, $CHCl_3$)

IR (KBr, $cm^{-1}$): 1760, 1740, 1690

UV ($\lambda_{max}^{EtOH}$): 210 nm, 238 nm

NMR ($CDCl_3$, δ ppm): 1.28 (5′-9′-$CH_2$—)

MS (m/e): 670, 628, 586, 458, 434, 416

| Elemental analysis: | C | H |
|---|---|---|
| Found | 64.24 | 6.88%; |
| Calcd. | 64.46 | 6.91%. |

(10) n=5

Form: amorphous

SR: $[\alpha]_D^{24}$ +6.47° (c=0.17, $CHCl_3$)

IR (KBr, $cm^{-1}$): 1760, 1740, 1680

UV ($\lambda_{max}^{EtOH}$): 210 nm, 238 nm

NMR ($CDCl_3$, δ ppm): 1.28 (5′-8′-$CH_2$—)

MS (m/e): 656, 614, 572, 444, 420, 402

| Elemental analysis: | C | H |
|---|---|---|
| Found | 63.90 | 6.77%; |
| Calcd. | 64.01 | 6.57%. |

(11) n=4

Form: amorphous

IR (KBr, $cm^{-1}$): 1760, 1750, 1730, 1680

UV ($\lambda_{max}^{EtOH}$): 210 nm, 238 nm

NMR ($CDCl_3$, δ ppm): 1.28 (5′-7′-$CH_2$—)

| Elemental analysis: | C | H |
|---|---|---|
| Found | 63.36 | 6.56%; |
| Calcd. | 63.54 | 6.59%. |

(12) n=3

Form: amorphous

IR (KBr, $cm^{-1}$): 1760, 1730, 1680

UV ($\lambda_{max}^{EtOH}$): 210 nm, 238 nm

NMR ($CDCl_3$, δ ppm): 1.28 (5′-6′-$CH_2$—)

| Elemental analysis: | C | H |
|---|---|---|
| Found | 62.91 | 6.33%; |
| Calcd. | 63.06 | 6.37%. |

(13) n=2

Form: amorphous

IR (KBr, $cm^{-1}$: 1760, 1750, 1730, 1680

UV ($\lambda_{max}^{EtOH}$): 210 nm, 238 nm

NMR ($CDCl_3$, δ ppm): 1.28 (5′-$CH_2$—)

(iii) Compound, IIa ($R_1$=$CH_3CO$—)

General formula

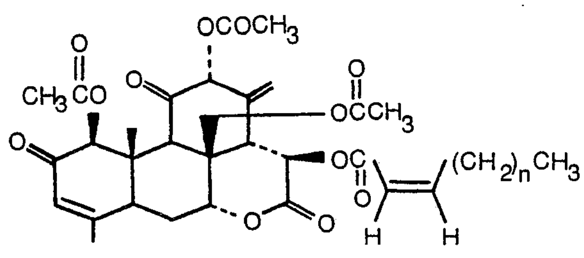

(1) n=7

Form: amorphous

SR: $[\alpha]_D^{26}$ −22.3° (c=0.39, $CHCl_3$)

IR (KBr, $cm^{-1}$): 1750, 1690

UV ($\lambda_{max}^{EtOH}$): 213 nm ($\varepsilon$ = 19200), 240 nm(sh) ($\varepsilon$ = 12800)

NMF. ($CDCl_3$, $\delta$ ppm): 0.84 (3H, t, terminal—$CH_3$); 1.42 (3H, s, 10-$CH_3$); 1.93 (3H, s, 4-$CH_3$);

2.00, 2.07, 2.13 (each 3H, s, $-O\overset{\overset{O}{\|}}{C}-CH_3$);

3.08 (1H, brd, 5-H); 3.28 (1H, d, J=12Hz, 14-H); 3.56, 4.56 (each 1H, d, J=12Hz, —$CH_2O$—); 3.67 (1H, s, 9-H); 4.70 (1H, t, 7-H); 5.20 (1H, s, 12-H);

5.27, 5.47 (each 1H, s, $=\!\!\genfrac{}{}{0pt}{}{/H}{\backslash H}$);

5.40 (1H, s, 1-H); 5.76 (1H, dt, J=12Hz, 1Hz, 2′-H); 6.02 (1H, brs, 3-H); 6.24 (1H, d, J=12Hz, 15-H); 6.36 (1H, dt, J=12Hz, 8Hz, 3′-H)

MS (m/e): 684 ($M^+$), 642, 600, 458, 416

| Elemental analysis: | C | H |
|---|---|---|
| Found | 65.01 | 7.17%; |
| Calcd. | 64.89 | 7.07%. |

(2) n=10

Form: amorphous

SR: $[\alpha]_D^{26}$ −23.8° (c=0.24, $CHCl_3$)

IR (KBr, $cm^{-1}$): 1750, 1690

UV ($\lambda_{max}^{EtOH}$): 213 nm ($\varepsilon$ = 19600), 240 nm (sh) ($\varepsilon$ = 12600)

NMR ($CDCl_3$, $\delta$ ppm): 0.84 (3H, t, terminal-$CH_3$); 1.42 (3H, s, 10-$CH_3$); 1.93 (3H, s, 4-$CH_3$);

2.00, 2.07, 2.13 (each 3H, s, $-O\overset{\overset{O}{\|}}{C}-CH_3$);

3.08 (1H, brd, 5-H); 3.28 (1H, d, J=12Hz, 14-H); 3.56, 4.56 (each 1H, d, J=12Hz, —$CH_2O$—); 3.67 (1H, s, 9-H); 4.70 (1H, t, 7-H); 5.20 (1H, s, 12-H);

5.27, 5.47 (each 1H, s, $=\!\!\genfrac{}{}{0pt}{}{/H}{\backslash H}$);

5.40 (1H, s, 1-H); 5.76 (1H, dt, J=12Hz, 1Hz, 2′-H); 6.02 (1H, brs, 3-H); 6.24 (1H, d, J=12Hz, 15-H); 6.36 (1H, dt, J=12Hz, 8Hz, 3′-H)

MS (m/e): 726 ($M^+$), 684, 642, 458, 416

| Elemental analysis: | C | H |
|---|---|---|
| Found | 66.29 | 7.32%; |
| Calcd. | 66.10 | 7.49%. |

(iv) Compound IIb
General formula

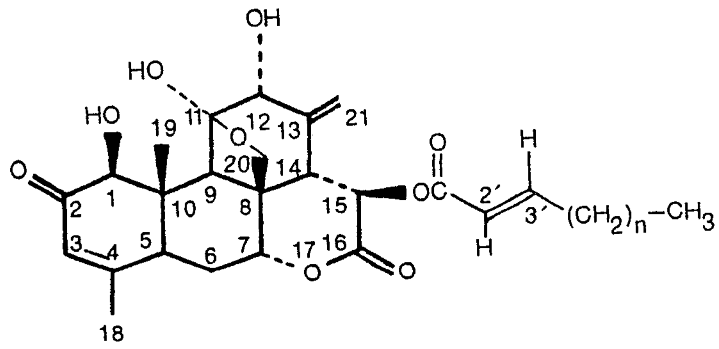

(1) n=14
Form: amorphous
SR: $[\alpha]_D^{27}$ +56.1° (c=0.13, $CHCl_3$)
IR (KBr, $cm^{-1}$): 3250, 1760, 1730, 1670
UV ($\lambda_{max}^{EtOH}$): 214 nm, 238 nm
NMR ($CDCl_3$, δ ppm): 0.90 (terminal-$CH_3$); 1.23 (3H, s, 10-$CH_3$); 1.28 (brs, 5′-17′$CH_2$); 2.04 (3H, s, 4-$CH_3$); 3.08 (1H, s, 9-H); 3.55, 3.93 (1H, d, J=8Hz, —$CH_2O$—); 4.07 (1H, s, 12-H); 4.21 (1H, s, 1-H); 4.63 (1H, t, 7-H); 5.87 (1H, dt, J=16Hz, 1Hzx, 2′-H);

5.18, 5.36 (1H, s, 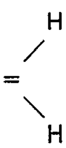 );

5.73 (1H, d, J=11Hz, 15-H); 6.16 (1H, brs, 3-H); 7.06 (1H, dt, J=16Hz, 7Hz, 3′-H)
MS (m/e): 656, 638, 612, 568

| Elemental analysis: | C | H |
|---|---|---|
| Found | 68.68 | 8.33%; |
| Calcd. | 68.76 | 8.34%. |

(2) n=13
Form: amorphous
IR (KBr, $cm^{-1}$): 3300, 1760, 1730, 1670
UV ($\lambda_{max}^{EtOH}$): 214 nm, 238 nm
NMR ($CDCl_3$, δ ppm): 1.28 (brs, 5′-16′-$CH_2$—)

| Elemental analysis: | C | H |
|---|---|---|
| Found | 69.01 | 8.45%; |
| Calcd. | 69.13 | 8.47%. |

(3) n=12
Form: amorphous
SR: $[\alpha]_D^{23}$ +50.0° (c=0.15, $CHCl_3$)
IR (KBr, $cm^{-1}$): 3300, 1760, 1730, 1670
UV ($\lambda_{max}^{EtOH}$): 214 nm, 238 nm
NMR ($CDCl_3$, δ ppm): 1.28 (brs, 5′-15′-$CH_2$—)
MS (m/e): 628, 610, 600, 586, 568, 558, 556, 540
High resolution MS: Found m/e 628.3595
(Calcd. for $C_{36}H_{52}O_9$, m/e 628.3610)

| Elemental analysis: | C | H |
|---|---|---|
| Found | 68.68 | 8.33%; |
| Calcd. | 68.76 | 8.34%. |

(4) n=11

Form: amorphous
IR (KBr, $cm^{-1}$): 3310, 1760, 1730, 1670
UV ($\lambda_{max}^{EtOH}$): 214 nm, 238 nm
NMR ($CDCl_3$, δ ppm): 1.28 (brs, 5'-14'-$CH_2$—)

| Elemental analysis: | C | H |
|---|---|---|
| Found | 68.19 | 8.18%; |
| Calcd. | 68.38 | 8.20%. |

(5) n=10

Form: amorphous
SR: $[\alpha]_D^{23}$ +38.2° (c=0.11, $CHCl_3$)
IR (KBr, $cm^{-1}$): 3320, 1760, 1730, 1670
UV ($\lambda_{max}^{EtOH}$): 214 nm, 238 nm
NMR ($CDCl_3$, δ ppm): 1.28 (5'-13'-$CH_2$—)
MS (m/e): 600, 582, 572, 558, 516, 512, 434, 416

| Elemental analysis: | C | H |
|---|---|---|
| Found | 67.81 | 8.07%; |
| Calcd. | 67.98 | 8.05%. |

(6) n=9

Form: amorphous
SR: $[\alpha]_D^{25}$ +37.9° (c=0.28, $CHCl_3$)
IR (KBr, $cm^{-1}$): 3300, 1760, 1730, 1670
UV ($\lambda_{max}^{EtOH}$): 214 nm, 238 nm
NMR ($CDCl_3$, δ ppm): 1,28 (5'-12'-$CH_2$—)

| Elemental analysis: | C | H |
|---|---|---|
| Found | 67.40 | 7.87%; |
| Calcd. | 67.55 | 7.90%. |

(7) n=8

Form: amorphous
SR: $[\alpha]_D^{23}$ +49.2° (c=0.12, $CHCl_3$)
IR (KBr, $cm^{-1}$): 3300, 1760, 1730, 1670
UV ($\lambda_{max}^{EtOH}$): 214 nm, 238 nm
NMR ($CDCl_3$, δ ppm): 1.28 (5'-11'-$CH_2$—)
High resolution MS: Found m/e 572.2940
(Calcd. for $C_{32}H_{44}O_9$, m/e 572. 2983)

| Elemental analysis: | C | H |
|---|---|---|
| Found | 66.98 | 7.74%; |
| Calcd. | 66.61 | 7.74%. |

(8) n=7

Form: amorphous
SR: $[\alpha]_D^{23}$ +36.0° (c=0.15, $CHCl_3$)
IR (KBr, $cm^{-1}$): 3250, 1760, 1730, 1670
UV ($\lambda_{max}^{EtOH}$): 214 nm, 238 nm
MS (m/e): 558, 540, 530, 516, 486, 470
High resolution MS: Found m/e 558.2807
(Calcd. for $C_{31}H_{42}O_9$, m/e 558.2825)

| Elemental analysis: | C | H |
|---|---|---|
| Found | 66.45 | 7.58%; |
| Calcd. | 66.65 | 7.58%. |

(9) n=6

Form: amorphous

SR: $[\alpha]_D^{27}$ + 53.0° (c=0.201, $CHCl_3$)

IR (KBr, $cm^{-1}$): 3250, 1750, 1720, 1660

UV ($\lambda_{max}^{EtOH}$): 214 nm, 238 nm

NMR ($CDCl_3$, δ ppm): 1.28 (brs, 5'-9'-$CH_2$—)

MS (m/e): 544, 526, 516, 502, 498, 472, 458, 456

High resolution MS: Found m/e 544.2694

(Calcd. for $C_{30}H_{40}$, m/e 544.2672)

| Elemental analysis: | C | H |
|---|---|---|
| Found | 66.07 | 7.39%; |
| Calcd. | 66.16 | 7.40%. |

(10) n=5

Form: amorphous

SR: $[\alpha]_D^{23}$ + 45,0° (c=0.16, $CHCl_3$)

IR (KBr, $cm^{-1}$): 3300, 1760, 1730, 1660

UV ($\lambda_{max}^{EtOH}$): 214 nm, 238 nm

NMR ($CDCl_3$, δ ppm): 1.28 (brs, 5'-8'-$CH_2$—)

MS (m/e): 530, 512, 502, 488, 458, 442

High resolution MS: Found m/e 530.2510

(Calcd. for $C_{29}H_{38}O_9$, m/e 530.2513)

| Elemental analysis: | C | H |
|---|---|---|
| Found | 65.50 | 7.19%; |
| Calcd. | 65.64 | 7.22%. |

(11) n=4

Form: amorphous

IR (KBr, $cm^{-1}$): 3300, 1760, 1730, 1660

UV ($\lambda_{max}^{EtOH}$): 214 nm, 238 nm

NMR ($CDCl_3$, δ ppm): 1.28 (brs, 5'-7'-$CH_2$—)

| Elemental analysis: | C | H |
|---|---|---|
| Found | 64.93 | 6.95%; |
| Calcd. | 65.11 | 6.98%. |

(12) n=3

Form: amorphous

IR (KBr, $cm^{-1}$): 3310, 1760, 1730, 1660

UV ($\lambda_{max}^{EtOH}$): 214 nm, 238 nm

NMR ($CDCl_3$, δ ppm): 1.28 (brs, 5'-6'-$CH_2$—)

| Elemental analysis: | C | H |
|---|---|---|
| Found | 64.29 | 6.79%; |
| Calcd. | 64.53 | 6.82%. |

(13) n=2

Form: amorphous

IR (KBr, $cm^{-1}$): 3300, 1750, 1730, 1660

UV ($\lambda_{max}^{EtOH}$): 214 nm, 238 nm

NMR ($CDCl_3$, δ ppm): 1.28 (brs, 5'-$CH_2$—)

| Elemental analysis: | C | H |
|---|---|---|
| Found | 63.78 | 6.58%; |
| Calcd. | 63.28 | 6.60%. |

(v) Compound IIb

General formula

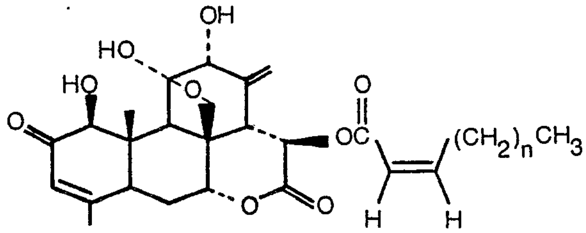

(1) n=7

Form: amorphous

SR: $[\alpha]_D^{26}$ +50.0° (c=0.10, $CHCl_3$)

IR (KBr, $cm^{-1}$): 3300, 1750, 1720, 1660

UV ($\lambda_{max}^{EtOH}$): 216 nm (ε = 18700), 240 nm (sh) (ε = 10200)

NMR ($CDCl_3$, δ ppm): 0.84 (3H, t, terminal-$CH_3$); 1.16 (3H, s, 10-$CH_3$); 2.00 (3H, s, 4-$CH_3$); 3.02 (1H, s, 9-H); 3.50, 3.89 (1H, d, J=8Hz, —$CH_2O$—); 4.01 (1H, s, 12-H); 4.12 (1H, s, 1-H); 4.59 (1H, t, 7-H);

5.13, 5.32 (each 1H, s, = 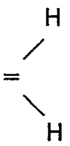 );

5.66 (1H, d, J=11Hz, 15-H); 5.80 (1H, dt, J=12Hz, 2′-H); 6.12 (1H, brs, 3-H); 6.34 (1H, dt, J=12Hz, 8Hz, 3′-H)

MS (m/e): 558, 540, 530, 514, 485, 470

High resolution MS: Found m/e 558.2841

Calcd. for $C_{31}H_{42}O_9$, m/e 558.2828

(2) n=10

Form: amorphous

SR $[\alpha]_D^{26}$ + 41.3° (c=0.15, $CHCl_3$)

IR (KBr, $cm^{-1}$): 3300, 1750, 1720, 166;

UV ($\lambda_{max}^{EtOH}$): 217 nm (ε = 17900), 240 nm (ε = 11300)

NMR ($CDCl_3$, δ ppm): 0.84 (3H, t, terminal-$CH_3$); 1.16 (3H, s, 10-$CH_3$); 2.00 (3H, s, 4-$CH_3$); 3.02 (1H, s, 9-H); 3.50, 3.89 (1H, d, J=8Hz, —$CH_2O$—); 4.01 (1H, s, 12-H); 4.12 (1H, s, 1-H); 4.59 (1H, t, 7-H);

5.13, 5.32 (each 1H, s, = 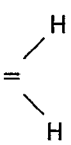 );

5.66 (1H, d, J=11Hz, 15-H); 5.80 (1H, dt, J=12Hz, 2′-H); 6.12 (1H, brs, 3-H); 6.34 (1H, dt, J=12Hz, 8Hz, 3′-H)

MS (m/e): 600 ($M^+$), 528, 572, 556, 527, 512

High resolution MS: Found m/e 600.3298

Calcd. for $C_{34}H_{48}O_9$, m/e 600.3298

## Preparation of Compounds

As hereinbefore described, the compounds I, IIa and IIb can be derived from ailanthone III as follows:

### Example 1

Ailanthone triacetate (1,12,20-triacetyl ailanthone), IV 87.4 g (0.23 M) of ailanthone was dissolved in 870 ml of anhydrous pyridine and 1700 ml of acetic anhydride was added to the solution. The reaction mixture was allowed to stand for 20 hours at room temperature and then evaporated *in vacuo* to precipitate a crystalline substance. This crystalline substance was recrystallized from methanol to give 108.6 g (yield 93%) of compound IV as colorless needles. This compound was in agreement with known ailanthone triacetate in view of its physico-chemical data and further it did not show any melting point depression in the mix-melting test with the authentic sample of compound IV.

### Example 2

16-hydroxyailanthone-triacetate(1,12,20-triacetyl-ailanthone-16-ol), V

15 g (30 mM) of ailanthone triacetate was dissolved in the mixed solvent containing 1.5 l of each of tetrahydrofurane and ethanol. To the solution, 2.25 g (60 mM) of sodium borohydride was added under ice-cooling. The reaction mixture was stirred for 2 hours under ice-cooling and thereafter a saturated aqueous ammonium chloride solution was added. Then, water was added so as to redissolve the formed precipitates and the organic solvent was evaporated *in vacuo*. The remaining liquid was extracted five times with methylene chloride and fractioned solvent layers were washed twice with water, then washed with saturated salt solution and finally dried over anhydrous magnesium sulfate. This dried extract was then evaporated *in vacuo* to give 14.7 g of the compound V as colorless powder. Yield: 98%

Form: amorphous
SR: $[\alpha]_D^{25}$ + 5.00° (c=0.14, $CHCl_3$)
IR (KBr, $cm^{-1}$): 3450, 1750, 1740, 1680
MS (m/e): $M^+$ 504, 462, 444, 420, 402, 378, 360
High resolution MS: Found m/e 504.2013
(Calcd. for $C_{26}H_{32}O_{10}$ m/e 504.1996)

| Elemental analysis: | C | H |
|---|---|---|
| Found | 61.76 | 6.39%; |
| Calcd. | 61.89 | 6.39%. |

Example 3

15,16-dehydroailanthone-triacetate(1,12,20-triacetyl-ailanthone-15-ene), VI

3.48 g (6.9 mM) of compound V was dissolved in 35 ml of pyridine and to the solution thus obtained 2.1 g (12.9 mM) of phosphorylchloride was added. The reaction mixture was refluxed for 5 minutes under argon atomsphere, followed by addition of ice in order to quench the reaction, and then pyridine was evaporated *in vacuo*. The remaining oily residue was diluted with water and extracted five times with ethyl acetate. The fractioned organic solvent layers were joined together and washed twice with saturated salt solution followed by drying over anhydrous sodium sulfate. The dried extract was condensed *in vacuo* to yield colorless crystals. The crystals were recrystallized from ethanol to give 2.01 g of the desired compound VI as colorless crystals. Yield: 60%

Form: colorless needles
mp: 186—187°C
SR: $[\alpha]_D^{25}$ −2.86° (c=0.21, $CHCl_3$)
IR (KBr, $cm^{-1}$): 1760, 1680
UV ($\lambda_{max}^{EtOH}$): 206 nm ($\varepsilon$ = 12000), 238 nm ($\varepsilon$ = 11400)
NMR ($CDCl_3$, $\delta$ ppm): 1.44, s (3H, 10-$CH_3$); 1.94, s (3H, 4-$CH_3$); 2.04, s (9H, —OAcX3); 3.24 d (1H, 5α-H); 3.44, t (1H, 14β-H); 3.68, s (1H, 9α-H);

3.62, d, J=12Hz 1H }
4.64, d, J=12Hz 1H } (—$CH_2$—OAc);

4.16, t (1H, 7β-H); 4.66, dd, J=7Hz, 3Hz (1H, 15-H); 5.15, 5 (1H, 12-H); 5.24, 5 (1H, 1-H);

5.24, s, 1H }
5.34, s, 1H }
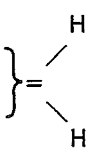
;

5.98q, J=1Hz (1H, 3-H); 6.41, dd, J=3Hz, 7Hz (1H, 16-H)
MS (m/e): $M^+$ 486, 444, 426, 402, 384, 360, 342

| Elemental analysis: | C | H |
|---|---|---|
| Found | 64.08 | 6.20%; |
| Calcd. | 64.19 | 6.22%. |

Example 4

15,16-dihydroxyailanthone-triacetate (1,12,20-triacetylailanthone-15,16-diol), VII

First, a solution of an oxidizing agent was prepared in the following manner: 5.06 g (43 mM) of N-methylmorpholine N-oxide was dissolved in a mixture containing 72.5 ml of water and 29.0 ml of acetone. To this solution, 21.8 mg (0.0857 mM) of osmium tetroxide in 10 ml of t-butanol was added to give the desired solution of the oxidizing agent.

To the above oxidized solution, 13.45 g (27.6 mM) of 1,12,20-triacetylailanthone-15-ene VI was added and the resulting solution was diluted with 145 ml of acetone. The reaction mixture was stirred at room

temperature for 24 hours, followed by further addition of 290 mg (2.79 mM) of sodium hydrogen sulfite. Then water was poured into the mixture, whereby the solution was homogenized. The solution thus obtained was then condensed *in vacuo* and the residue was adjusted to pH 2 with 2N HCl. The residue was then saturated with salt, and extracted 5 times with ethyl acetate and the combined extract was then dried over anhydrous sodium sulfate. The dried extract was then evaporated *in vacuo* to give the diol compound VII as colorless powder. (This compound was a mixture of C-16 stereoisomers so that it was impossible to be crystallized).

The above powder was then chromatographed on 25 g of silica gel (CC-7) (prepared by Mallinckrodt Co.) and eluted with ethyl acetate to obtain 13 g of pure product. Yield: 90%.

Form: amorphous
SR: $[\alpha]_D^{25}$ + 3.81° (c=0.21, $CHCl_3$)
IR ($cm^{-1}$): 3450, 1760, 1740, 1680
MS (m/e): $M^+$ 520, 502, 478, 460, 436, 418, 400, 376, 358
High resolution MS: Found m/e 520.1958
Calcd. for $C_{26}H_{32}O_{11}$ m/e 520.1943

| Elemental analysis: | C | H |
|---|---|---|
| Found | 59.82 | 6.21%; |
| Calcd. | 59.99 | 6.20%. |

Example 5

15β-hydroxyailanthone-triacetate(1,12,20-triacetyl-ailanthone-15β-ol), I

17.4 g (33.4 mM) of diol compound VII was dissolved in 850 ml of anhydrous acetonitrile, followed by the addition of 52.5 g (421 mM) of activated silver oxide. The mixture was refluxed for 1 hour. After cooling to room temperature, the silver salt was removed from the mixture with cerite. Evaporation of the solvent from the reaction mixture gave 22.6 g of an oily substance. This substance was chromatographed on 300 g of above-described silica gel (CC-7) and eluted with the mixture of ethyl acetate and chloroform (4:6 by volume). The eluate was condensed and the residue was recrystallized from acetone-ether mixture to give 10.4 g of 1,12,20-triacetylailanthone-15β-ol I. The overall yield of the compound I from compound IV was 31.7%.

Example 6

15β-hydroxyailanthone triacetate-α,β-unsaturated carboxylic acid esters, IIa

The compound IIa of the present invention can be prepared from the above compound I by the following general procedure.

1.24 equivalent of a $C_5$—$C_{18}$ α,β-unsaturated straight carboxylic acid and 1.50 equivalent of 1-ethyl-2-fluoropyridinium tetrafluoroborate or 1-methyl-2-fluoropyridinium tosylate were dissolved in anhydrous methylene chloride and 4.87 equivalent of cesium fluoride was added thereto. This solution was then stirred for 30 minutes at room temperature. To this solution, 1.00 g (1.93 mM) of compound I (15β-hydroxyailanthone-triacetate was added in one portion. The mixture was then further stirred at room temperature for 20 hours and the reaction was stopped.

The reaction mixture was then extracted three times with methylene chloride. The combined extract was then washed twice with saturated sodium hydrogen carbonate solution and further washed twice with saturated salt solution, and then dried over anhydrous magnesium sulfate, followed by condensation to remove the solvent. The remaining oily substance was then chromatographed on CC-7 silica gel (as described above) and eluted with a mixture of benzene-ethyl acetate (9:11 by volume) to obtain the compound IIa as a colorless oily substance. The following Table I shows the yields of several compounds IIa obtained according to this general procedure.

TABLE 1

| Type of aliphatic acid | Amount of aliphatic acid (mg) | Yield of IIa mg (%) |
|---|---|---|
| trans-2-octadecenoic acid | 677 | 1.16 (77) |
| trans-2-heptadecenoic acid | 643 | 1.10 (74) |
| trans-2-hexadecenoic acid | 610 | 1.02 (70) |
| trans-2-pentadecenoic acid | 576 | 1.07 (75) |
| trans-2-tetradecenoic acid | 542 | 1.18 (84) |
| trans-2-tridecenoic acid | 509 | 1.26 (92) |
| trans-2-dodecanoic acid | 475 | 1.28 (95) |
| trans-2-undecenoic acid | 442 | 1.25 (95) |
| trans-2-decenoic acid | 408 | 1.16 (90) |
| trans-2-nonenoic acid | 374 | 1.04 (89) |
| trans-2-octenoic acid | 341 | 1.24 (93) |
| trans-2-heptenoic acid | 307 | 1.21 (95) |
| trans-2-hexenoic acid | 274 | 1.19 (95) |
| trans-2-pentanoic acid | 240 | 1.04 (90) |
| cis-2-undecenoic acid | 356 | 0.978 (74) |
| cis-2-tetradecenoic acid | 436 | 1.083 (74) |

Example 7

15ß-hydroxyailanthone-α,ß-unsaturated carboxylic acid esters, IIb.

The compound IIb can be prepared from the above compound IIa by the following general procedure.

1.0 mM of the compound IIa obtained according to Example 6 was dissolved in a methanolic solution of 0.01 N potassium hydroxide. The resulting solution was then stirred for 2 hours under $N_2$ atmosphere. The reaction mixture was then adjusted to pH 4—5 with 1N HCl and thereafter the solvent was distilled off. The residue was then extracted three times with methylene chloride after the addition of water. The fractionated methylene chloride layers were washed twice with saturated aqueous saline and dried over anhydrous magnesium sulfate. The solvent was removed from the dried extract *in vacuo* and the residue thus obtained was chromatographed on silica gel (Kiesel gel-60, Merck, 70—230 mesh ASTM) and then eluted with the mixture of ethyl acetate-hexane (5:2 by volume) to give a pure compound IIb. In addition, the mixture of monoacetate and diacetate fractionated by the above chromatography was re-acetylated with ten times amount of pyridine and twenty times amount of acetic anhydride at room temperature for 48 hours, and then hydrolyzed as in the above. Thus, the desired compound IIb can further be recovered.

The following Table 2 shows the yields of several compounds IIb obtained according to this general procedure.

TABLE 2

| Type of starting compound IIa | Yield of IIb (%) |
|---|---|
| trans-2-octadecenoate | 40 |
| trans-2-heptadecenoate | 42 |
| trans-2-hexadecenoate | 43 |
| trans-2-pentadecenoate | 40 |
| trans-2-tetradecenoate | 47 |
| trans-2-tridecenoate | 49 |
| trans-2-dodecenoate | 50 |
| trans-2-undecenoate | 51 |
| trans-2-decenoate | 54 |
| trans-2-nonenoate | 50 |
| trans-2-octenoate | 52 |
| trans-2-heptenoate | 44 |
| trans-2-hexenoate | 33 |
| trans-2-pentenoate | 34 |
| cis-2-undecenoate | 40 |
| cis-2-tetradecenoate | 41 |

Antineoplastic Activity of the Compounds of Present Invention.

*Materials*

Animals: Mouse ($BDF_1$, female, 4—5 weeks, average body weight: 18 g)

Cells: Mouse lymphocytic leucemia p388

Medicine: Compounds IIb (n = 5 (trans), 6 (trans), 7 (trans), 8 (trans), 9 (trans), 10 (trans), 12 (trans), 14 (trans), 7 (cis) and 10 (cis).

*Method*

$10^6$ cells of mouse lymphocytic leucemia p388 were intrapenetreally injected to $BDF_1$ mouse (6 mice per group). From the 2nd day after the injection, the destined amount of the medicine as shown in following Table 3 was administered for 5 days continuously. Thereafter, the surviving percentages (ILS) of the administered mice were calculated by the following equations.

$$ILS = \frac{\text{Mean survival time of the administered group}}{\text{Mean survival time of the control group}} \times 100 - 100\%$$

TABLE 3

| Dose (mg/kg) / Medicine IIb | 30 | 10 | 5 | 3 | 1 | 0.5 |
|---|---|---|---|---|---|---|
| n = 5 (trans) | 84 | 55 | 37 | — | — | — |
| n = 6 (trans) | 100 | 54 | 50 | — | — | — |
| n = 7 (trans) | 80 | 74 | 69 | — | — | — |
| n = 8 (trans) | 84 | 65 | 63 | — | — | — |
| n = 9 (trans) | 86 | 64 | 46 | — | — | — |
| n = 10 (trans) | 102 | 67 | 47 | — | — | — |
| n = 12 (trans) | 78 | 51 | 45 | — | — | — |
| n = 14 (trans) | 47 | 6 | 6 | — | — | — |
| n = 7 (cis) | 66 | 54 | 39 | — | — | — |
| n = 10 (cis) | 64 | 56 | 34 | — | — | — |
| control* | — | — | — | — | 83 | — |

* Mitomycin C was used as the positive control

As shown by the above Table 3, the compound IIb is markedly effective against mouse lymphocytic leucemia p338. Especially, the compounds of n = 6 (trans) and n = 10 (trans) are so effective that they can prolong the life of the administered group as much as twice or more as compared to that of the control group and far more effective than that of known mitomycin C adminsitered group. This fact will give a preferably aspect to the use of the compound of this invention as a novel antitumor agent.

While the invention has been described in detail and with reference to specific embodiment thereof, it will be apparent to one skilled in the art that various changes and modifications can be made therein without departing from the spirit and scope thereof.

**Claims**

1. Ailanthone derivatives represented by the formula:

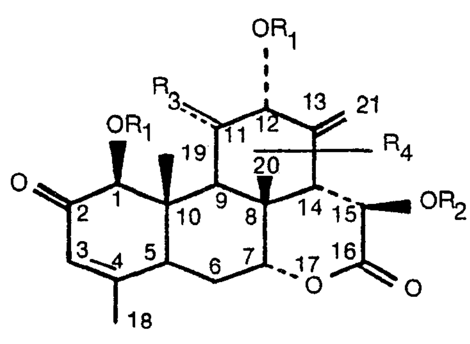

wherein $R_1$ is hydrogen, an acetyl, chloroacetyl, or benzoyl group, $R_2$ is a $C_5$—$C_{18}$ α,ß-unsaturated straight chain acyl group, $R_3$ is oxygen or a hydroxyl group and $R_4$ is oxygen connecting to $C_{11}$—$C_{20}$ carbon atom, or acetyloxy, chloroacetyloxy, or benzoyloxy group connecting to $C_{20}$ carbon atom of the nucleus, with the proviso, that when $R_4$ is oxygen, then $R_3$ is a hydroxyl group, and that when $R_4$ is an acyloxy group as defined above, then $R_3$ is oxygen.

2. An ailanthone derivative which is represented by the formula:

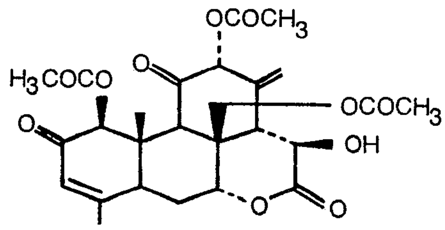

3. A method for preparing ailanthone derivatives represented by the following formula:

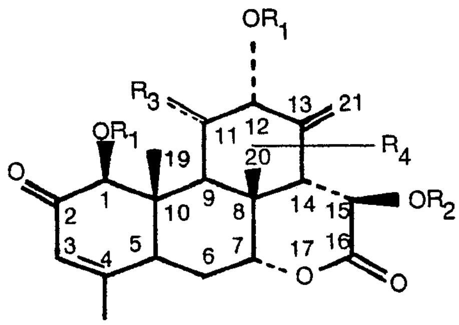

wherein $R_1$ is hydrogen, an acetyl, chloroacetyl, or benzoyl group, $R_2$ is a $C_5$—$C_{18}$ α,ß-unsaturated straight chain acyl group, $R_3$ is oxygen or a hydroxyl group and $R_4$ is oxygen connecting to $C_{11}$—$C_{20}$ carbon atom, or acetyloxy, chloroacetyloxy, or benzoyloxy group connecting to $C_{20}$ carbon atom of the nucleus, with the proviso, that when $R_4$ is oxygen, then $R_3$ is a hydroxyl group, and that when $R_4$ is an acyloxy group as defined above, then $R_3$ is oxygen, which comprises reducing a C—16 carbonyl group of the triacyl ailanthone represented by the formula IV:

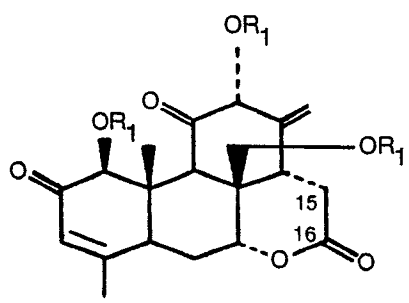

(IV)

wherein $R_1$ is an acyl group as defined above with a reducing agent to form a corresponding 16-hydroxy derivative represented by the formula V:

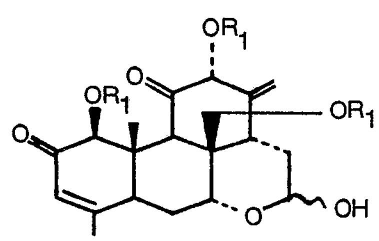

(V)

wherein $R_1$ is as defined above, dehydrating the resulting 16-hydroxy derivative with phosphoryl chloride or phosphorus pentoxide to form a corresponding triacyl 15(16)-unsaturated derivative represented by the formula VI:

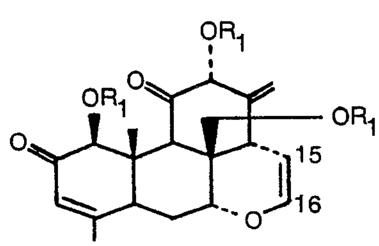

(VI)

wherein $R_1$ is as defined above, oxidizing the resulting triacyl 15(16)-unsaturated derivative with osmium tetroxide or osmium tetroxide and N-methylmorpholine-N-oxide to form a corresponding 15,16-diol derivative represented by the formula VII:

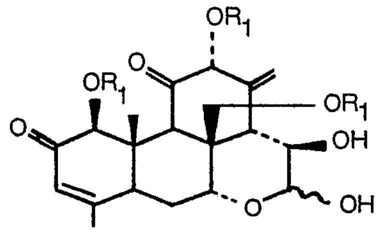

(VII)

wherein $R_1$ is as defined above, and oxidizing the 16-hydroxy group of the resulting 15,16-diol derivative with silver oxide to form a corresponding triacyl 15-hydroxy derivative represented by the formula I:

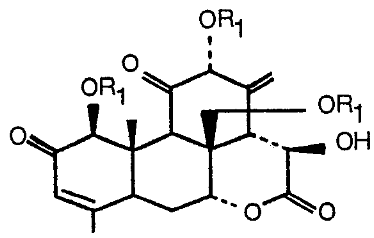

(I)

wherein $R_1$ is as defined above, and esterifying the $C_{15}$ hdyroxy group with a $C_5$—$C_{18}$ α,ß-unsaturated straight chain carboxylic acid to form a corresponding 15ß-carboxylic acid ester represented by the formula:

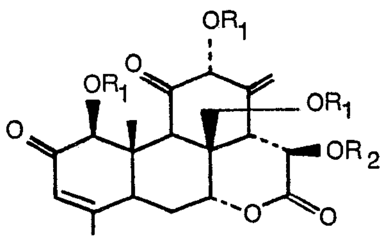

wherein $R_1$ is defined as above and $R_2$ is a $C_5$—$C_{18}$ α,ß-unsaturated straight chain acyl group and, optionally, hydrolyzing the resulting α,ß-unsaturated acyl group to obtain the corresponding trihydroxy compound represented by the formula:

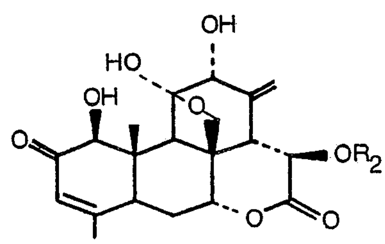

wherein $R_2$ is as defined above.

4. A pharmaceutic agent represented by the formula:

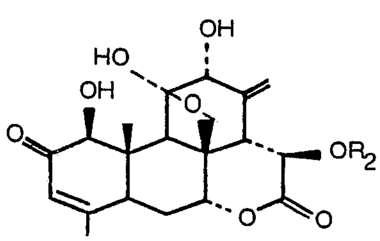

wherein $R_2$ represents a $C_5$—$C_{18}$ α,ß-unsaturated straight chain acyl group.

**Patentansprüche**

1. Ailanthon-Derivate entsprechend der Formel:

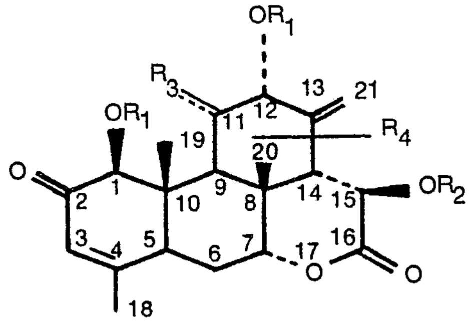

worin $R_1$ Wasserstoff, eine Acetyl-, Chloracetyl- oder Benzoylgruppe darstellt, $R_2$ eine $C_5$—$C_{18}$- α,ß-ungesättigte, geradkettige Acylgruppe bedeutet, $R_3$ Sauerstoff oder eine Hydroxylgruppe darstellt, und $R_4$ einen mit dem $C_{11}$—$C_{20}$-Kohlenstoffatom verbunden Sauerstoff oder eine Acetyloxy-, Chloracetyloxy- oder Benzoyloxygruppe in Verbindung mit dem $C_{20}$-Kohlenstoffatom des Kerns bedeutet, vorausgesetzt, dass, wenn $R_4$ Sauerstoff darstellt, $R_3$ eine Hydroxylgruppe bedeutet, und wenn $R_4$ eine Acyloxygruppe, wie vorstehend definiert, darstellt, $R_3$ Sauerstoff bedeutet.

2. Ailanthon-Derivat entsprechend der Formel:

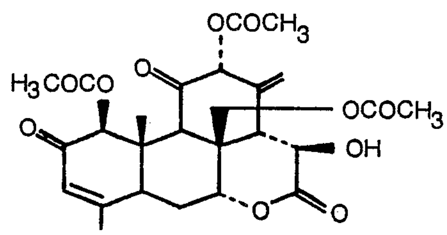

3. Verfahren zur Herstellung von Ailanthon-Derivaten entsprechend der folgenden Formel

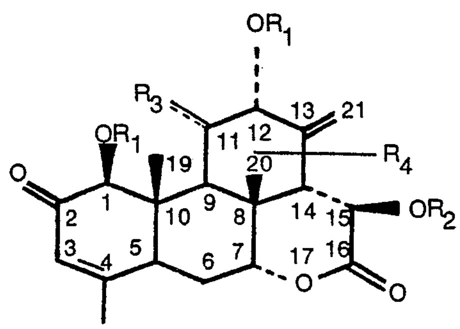

worin $R_1$ Wasserstoff, eine Acetyl-, Chloroacetyl- oder Benzoylgruppe darstellt, $R_2$ eine $C_5$—$C_{18}$- α,ß-ungesättigte, geradkettige Acylgruppe bedeutet, $R_3$ Sauerstoff oder eine Hydroxylgruppe darstellt, und $R_4$ Sauerstoff, welcher an das $C_{11}$—$C_{20}$-Kohlenstoffatom bindet, oder eine Acetyloxy-, Chloracetyloxy- oder Benzoyloxygruppe, die an das $C_{20}$-Kohlenstoffatom des Kerns bindet, bedeutet, vorausgesetzt, dass wenn $R_4$ Sauerstoff darstellt, $R_3$ eine Hydroxylgruppe bedeutet, und wenn $R_4$ eine Acyloxygruppe, wie vorstehend definiert, darstellt, $R_3$ Sauerstoff bedeutet, gekennzeichnet durch Reduzieren einer C—16-Carbonylgruppe des Triacylailanthons entsprechend der Formel IV:

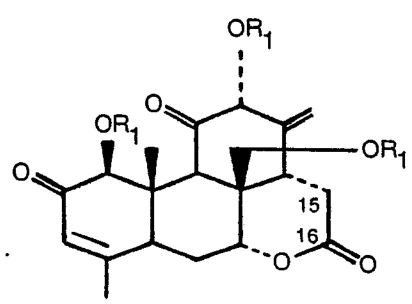

(IV)

worin $R_1$ eine Acylgruppe, wie vorstehend definiert, bedeutet, mit einem Reduktionsmittel zu dem entsprechenden 16—Hydroxy-Derivat gemäss Formel V:

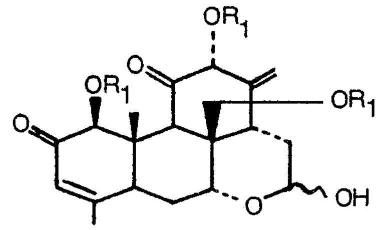

(V)

worin $R_1$ die vorstehend angegebene Bedeutung hat, Dehydratisieren des erhaltenen 16—Hydroxy-Derivates mit Phosphorylchlorid oder Phosphorpentoxid zum Erhalt eines korrespondierenden Triacyl-15(16)-ungesättigten Derivates entsprechend der Formel VI:

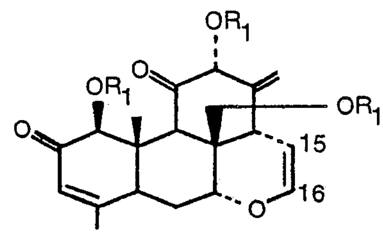

(VI)

worin $R_1$ die vorstehend angegebene Bedeutung hat, Oxidieren des erhaltenen Triacyl-15(16)-ungesättigten Derivates mit Osmiumtetroxid oder Osmiumtroxid und N-Methylmorpholin-N-oxid unter Bildung eines korrespondierenden 15,16-Diol-Derivates entsprechend Formel VII:

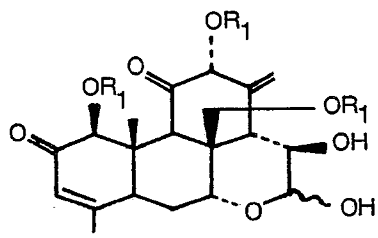

(VII)

worin $R_1$ die vorstehend angegebene Bedeutung hat, und Oxidieren der 16—Hydroxygruppe des erhaltenen 15,16-Diol-Derivatives mit Silberoxid unter Bildung eines entsprechenden Triacyl-15-hydroxy-Derivates gemäss Formel I:

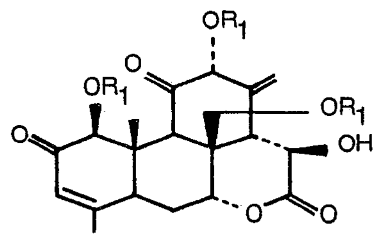

(I)

worin $R_1$ die vorstehend angegebene Bedeutung hat, und Verestern der $C_{15}$-Hydroxygruppe mit einer $C_5$-$C_{16}$- α.ß-ungesättigten, geradkettigen Carbonsäure unter Erhalt eines entsprechenden 15ß-Carbonsäureesters gemäss folgender Formel:

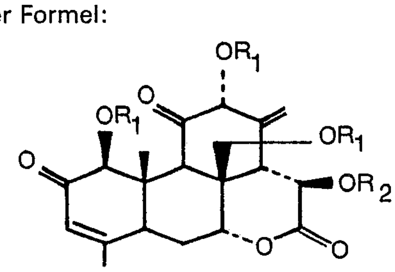

worin $R_1$ die vorstehend angegebene Bedeutung hat, und $R_2$ eine $C_5$—$C_{18}$- α,ß-ungesättigte, geradkettige Acylgruppe darstellt, und gegebenenfalls Hydrolysieren der erhaltenen α,ß-ungesättigten Acylgruppe unter Erhalt der entsprechenden Trihydroxyverbindung gemäss folgender Formel:

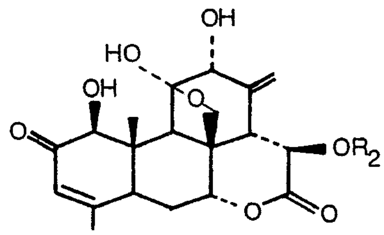

worin $R_2$ die vorstehend angegebene Bedeutung hat.

4. Pharmazeutisches Mittel gemäss folgender Formel:

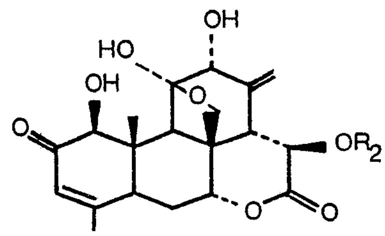

worin $R_2$ eine $C_5$—$C_{18}$- α,ß-ungesättigte, geradkettige Acylgruppe darstellt.

**Revendications**

1. Dérivés d'ailanthone, représentés par la formule:

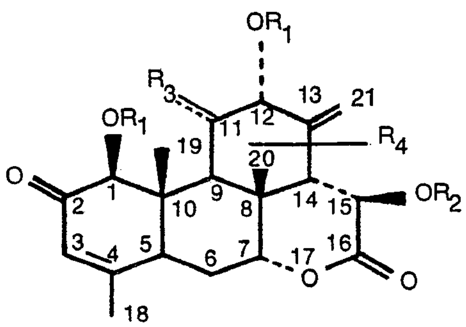

dans laquelle $R_1$ représente un atome d'hydrogène ou un groupe acétyle, chloroacétyle ou benzoyle; $R_2$ représente un groupe acyle linéaire en $C_5$—$C_{18}$, à insaturation en α,ß; $R_3$ représente un atome d'oxygène ou un groupe hydroxyle et $R_4$ représente un atome d'oxygène reliant les atomes de carbone $C_{11}$ et $C_{20}$, ou un groupe acétyloxy, chloroacétyloxy, ou benzoyloxy relié à l'atome de carbone en $C_{20}$ du noyau, à la condition que, quand $R_4$ représente un atome d'oxygène, $R_3$ est un groupe hydroxyle et lorsque $R_4$ représente un groupe acyloxy selon la définition ci-dessus, $R_3$ représente un atome d'oxygène.

2. Dérivé d'ailanthone, représenté par la formule:

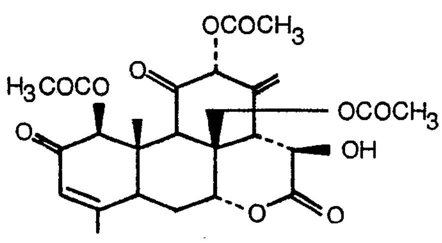

3. Procédé pour préparer des dérivés d'ailanthone représentés par la formule suivante:

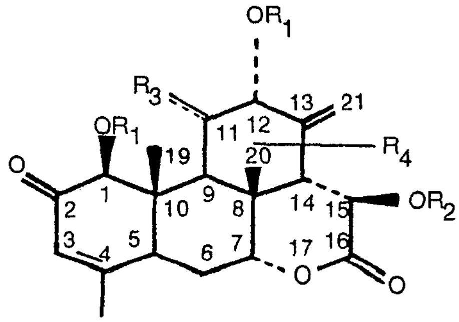

dans laquelle $R_1$ représente un atome d'hydrogène ou un groupe acétyle, chloracétyle ou benzoyle; $R_2$ représente un groupe acyle linéaire en $C_5$—$C_{18}$, à insaturation en α,ß; $R_3$ représente un atome d'oxygène ou un groupe hydroxyle et $R_4$ représente un atome d'oxygène reliant les atomes de carbone $C_{11}$ et $C_{20}$, ou un groupe acétyloxy, chloracétyloxy ou benzoyloxy relié à l'atome de carbone en $C_{20}$ dy noyau, à la condition que, quand $R_4$ représente un atome d'oxygène, $R_3$ est un groupe hydroxyle et lorsque $R_4$ représente un groupe acyloxy selon la définition ci-dessus, $R_3$ représente un atome d'oxygène, qui comprend la réduction d'un groupe carbonyle en $C_{16}$ de la triacylailanthone représentée par la formule IV:

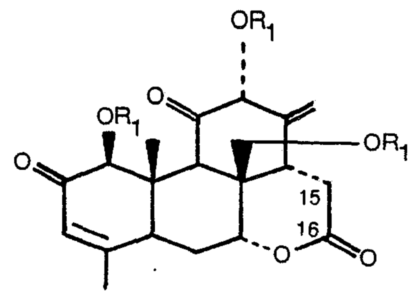

(IV)

dans laquelle $R_1$ est un groupe acyle selon la définition ci-dessus, avec un agent réducteur pour former un dérivé 16-hydroxy correspondant représenté par la formule V:

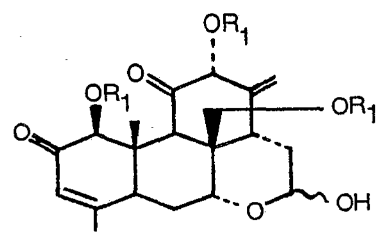

(V)

dans laquelle $R_1$ est tel que défini ci-dessus, la déshydratation du dérivé 17-hydroxy résultant par du chlorure de phosphoryle ou du pentoxyde de phosphore pour former un dérivé de composé triacylé insaturé en 15(16) correspondant, représenté par la formule VI:

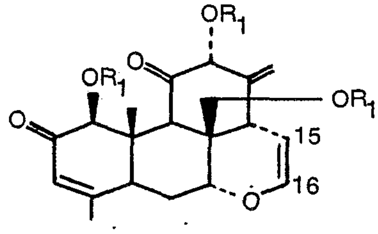

(VI)

dans laquelle $R_1$ est tel que défini ci-dessus, l'oxydation du dérivé triacylé insaturé en 15(16), par du tétroxyde d'osmium ou du tétroxyde d'osmium et du N-oxyde de N-méthylmorpholine pour former un 15,16-diol dérivé correspondant, représenté par la formule VII:

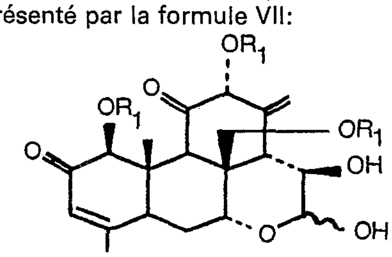

(VII)

dans laquelle $R_1$ est tel que défini ci-dessus, et l'oxydation du groupe 16-hydroxy du 15,16-diol dérivé résultant par de l'oxyde d'argent pour former un dérivé triacylé à groupe 15-hydroxy correspondant, représenté par la formule I:

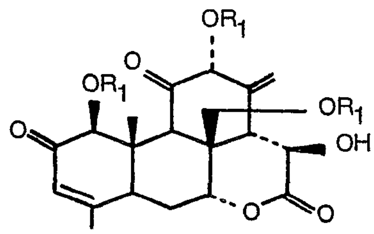

(I)

dans laquelle $R_1$ est tel que défini ci-dessus, et l'estérification du groupe hydroxy en $C_{15}$, par un acide carboxylique linéaire en $C_5$—$C_{18}$ insaturé en α,ß, pour former un ester d'acide carboxylique en 15ß correspondant, représenté par la formule:

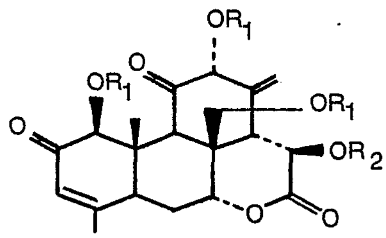

dans laquelle $R_1$ est tel que défini ci-dessus et $R_2$ représente un groupe acyle linéaire en $C_5$—$C_{18}$ insaturé en α,ß et, éventuellement, l'hydrolyse du groupe acyle insaturé en α,ß résultant pour obtenir le composé trihydroxylé correspondant représenté par la formule:

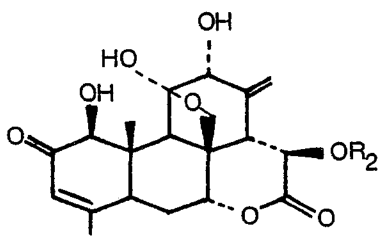

dans laquelle $R_2$ est tel que défini ci-dessus.

4. Produit pharmaceutique, représenté par la formule:

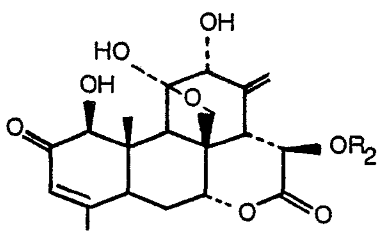

dans laquelle $R_2$ représente un groupe acyle linéaire en $C_5$-$C_{18}$ insaturé en α,ß.